# EUROPÄISCHE PATENTSCHRIFT

(11) **EP 0 917 882 B1**
(45) Veröffentlichungstag und Bekanntmachung des Hinweises auf die Patenterteilung: **28.05.2003**
(21) Anmeldenummer: 98119899.7
(22) Anmeldetag: 21.10.1998
(51) Int. Cl.: A61M 5/32, A61M 5/24, A61J 1/00, A61J 1/06

(54) **Spritze, insbesondere vorgefüllte Spritze oder Karpule**
Syringe particularly a prefilled syringe or ampoule
Seringue en particulier une seringue ou ampoule préremplie

(30) Priorität: 19.11.1997 DE 19751219
(43) Veröffentlichungstag der Anmeldung: 26.05.1999
(73) Patentinhaber: Arzneimittel GmbH Apotheker Vetter & Co. Ravensburg, D-88212 Ravensburg (DE)
(72) Erfinder: Vetter, Helmut, 88212 Ravensburg (DE); Otto, Thomas, 88250 Weingarten (DE)
(74) Vertreter: Dziewior, Joachim, Dipl.-Phys. Dr.

(56) Entgegenhaltungen:
- WO-A-94/03373
- WO-A-96/40037
- CH-A- 621 524
- FR-A- 1 139 441
- US-A- 2 906 423
- US-A- 5 320 603

## Beschreibung

Die Erfindung betrifft eine Spritze, insbesondere vorgefüllte Spritze, oder Karpule für medizinische Zwecke, mit einem Spritzenzylinder und gegebenenfalls einem darin angeordneten, mittels einer Kolbenstange verschiebbaren Spritzenkolben, ferner mit einem am kanülenseitigen Ende des Spritzenzylinders axial aufgesetzten Verschlußteil, das das kanülenseitige Ende des Spritzenzylinders umgreift, sowie mit einem Dichtungselement, das zwischen der Stirnfläche des Spritzenzylinders und dem Verschlußteil angeordnet ist und einerseits stirnseitig dem Spritzenzylinder und andererseits der inneren Stirnfläche des Verschlußteils anliegt, wobei das Verschlußteil mit einer zentralen Ausnehmung zum Durchstechen mit einer Kanüle versehen ist, sowie mit einer das Verschlußteil umschließenden Sicherungskappe, die formschlüssig auf das Verschlußteil aufgesetzt und in aufgesetztem Zustand auf dem Verschlußteil gehalten ist.

Bei bekannten Spritzen bzw. Karpulen dieser Art muß nach dem Entfernen der Sicherungskappe zunächst dafür gesorgt werden, daß auf dem Dichtungselement im Bereich der Ausnehmung haftende Verunreinigungen beim Durchstechen nicht mit ins Innere der Spritze bzw. der Karpule verschleppt werden. Dies bedeutet, daß die Oberfläche gereinigt werden muß, was nicht nur zeitaufwendig, sondern darüber hinaus auch umständlich ist, da die zu reinigende Oberfläche im Bodenbereich der Ausnehmung liegt. Ferner bedeutet dies, daß zusätzlich Mittel zur Reinigung bzw. Sterilisierung bereitgehalten werden müssen.

Die US-A-2 906 423 und die US-A-5 320 603 offenbaren die Verwendung einer Sicherungskappe mit einem darin angeordneten Dichtungsteil. Dieses muß jedoch nach dem Entfernen der Sicherungskappe jeweils separat entfernt werden, was die Gefahr der Verunreinigung des zu durchstechenden Dichtungselementes in sich birgt.

Der Erfindung liegt die Aufgabe zugrunde, eine Spritze bzw. Karpule der eingangs genannten Art so auszubilden, daß der zum Durchstechen mit einer Kanüle vorgesehene Bereich des Dichtungselements nach dem Entfernen der Sicherungskappe, also vor der Anwendung der Spritze bzw. der Karpule, keiner - sterilisierenden - Reinigung bedarf.

Diese Aufgabe wird erfindungsgemäß durch eine Spritze oder Karpule gemäß Anspruch 1 gelöst

Der durch die Erfindung erreichte Vorteil besteht im wesentlichen darin, daß das Dichtungsteil nach dem Aufsetzen der Sicherungskappe die von der Ausnehmung im Verschlußteil freigegebene Oberfläche verschließt, so daß diese von Verunreinigungen freigehalten wird. Das Aufsetzen der Sicherungskappe mit dem elastischen Dichtungsteil erfolgt dabei am Ende des Herstellungs- bzw. Konfektionierablaufs noch unter sterilen Bedingungen, so daß die freiliegende Oberfläche des Dichtungselements steril ist und von dem Dichtungsteil auch steril gehalten wird.

Das Dichtungsteil ist als im wesentlichen zylindrischer Stopfen ausgebildet, der in einem Sackloch der Sicherungskappe angeordnet ist und an seiner zum Dichtungselement weisenden Stirnseite eine entlang des Umfangs verlaufende Ringlippe aufweist. Der Stopfen ist dabei im Durchmesser zweckmäßigerweise an den Durchmesser der Ausnehmung angepaßt. Die Ringlippe gewährleistet hierbei besonders günstige Abdichtungseigenschaften.

Weiter ist im Rahmen der Erfindung vorgesehen, daß das Dichtungsteil an seiner dem Dichtungselement abgewandten Stirnseite einen radial vorstehenden Ringflansch aufweisen kann, der in eine Ringnut in der Wand des Sacklochs der Sicherungskappe vorsteht. Hierdurch ist ein sicherer Halt des Dichtungsteils in der Sicherungskappe gewährleistet.

In einer ersten Ausgestaltung der Erfindung, die vorzugsweise bei Spritzen Anwendung findet, trägt die Sicherungskappe einen über eine ringförmig umlaufende Sollbruchzone angeschlossenen Sicherungsring, der über eine Ringschulter im Rastsitz am Verschlußteil gehalten ist. Zum Gebrauch der Spritze kann dann die Sicherungskappe entlang der Sollbruchzone abgetrennt werden, wodurch sichergestellt ist, daß das Verschlußteil nach wie vor über den Sicherungsring gehalten wird. Im übrigen bildet die Sicherungskappe hierbei einen Originalitätsverschluß, wodurch der Benutzer erkennen kann, ob die Spritze bereits für den Gebrauch vorbereitet bzw. benutzt worden ist oder sich noch in unversehrtem Zustand befindet.

Bei einer zweiten Ausgestaltungsform der Erfindung, wie sie vorzugsweise bei Karpulen zur Anwendung kommt, bei denen das Verschlußteil von einer Bördelkappe gebildet ist, ist die Sicherungskappe zweckmäßigerweise von einer Schrumpffolie umhüllt. Diese Schrumpffolie stellt wiederum einen Originalitätsverschluß dar, dient im übrigen aber dazu, die Sicherungskappe auf dem Verschlußteil zu halten. Da bei marktüblichen Karpulen der durch das Verschlußteil bestimmte Außendurchmesser nicht vergrößert werden darf, um die Spritze bzw. Karpule in den gängigen Applikationssystemen anwenden zu können, muß vor der Injektion die Sicherungskappe abgenommen werden. Die Sicherungskappe kann daher nicht ohne weiters mit einrastenden Mitteln versehen werden, die ein Entfernen der Sicherungskappe erschweren würden. Auf diese Weise kann die Sicherungskappe im ganzen entfernt werden, sobald die Schrumpffolie gelöst wurde.

Im folgenden wird die Erfindung an in der Zeichnung dargestellten Ausführungsbeispielen erläutert; es zeigen:
- Fig. 1: eine Spritze in nur teilweiser Darstellung vor dem Aufsetzen der Sicherungskappe,
- Fig. 2: den Gegenstand nach Fig. 1, jedoch mit aufgesetzter Sicherungskappe,
- Fig. 3: den Gegenstand nach Fig. 2, jedoch vorbereitet zur Applikation,
- Fig. 4: eine der Fig. 1 entsprechende Darstellung, jedoch am Beispiel einer Karpule,
- Fig. 5: den Gegenstand nach Fig. 4, in der Fig. 2 entsprechendem Zustand,
- Fig. 6: den Gegenstand nach Fig. 4, bei der Vorbereitung zur Applikation.

Die in der Zeichnung dargestellte, für medizinische Zwecke vorgesehene Spritze, die insbesondere vorgefüllt in den Verkehr kommt, bzw. die Karpule besteht zunächst aus einem Spritzenzylinder 9, in dem - gegebenenfalls - ein mittels einer Kolbenstange verschiebbarer, in der Zeichnung jedoch nicht dargestellter Spritzenkolben angeordnet sein kann. Am kanülenseitigen Ende des Spritzenzylinders 9 ist ein Verschlußteil 3 axial aufgesetzt, das das kanülenseitige, hier flaschenhalsartig ausgebildete Ende des Spritzenzylinders 9 umgreift. Zwischen der Stirnfläche des Spritzenzylinders 9 und dem Verschlußteil 3 ist ein Dichtungselement 5 angeordnet, das einerseits stirnseitig dem Spritzenzylinder 9 und andererseits der inneren Stirnfläche des Verschlußteils 3 anliegt.

Das Verschlußteil 3 ist mit einer zentralen Ausnehmung 10 versehen, die zum Durchstechen des Verschlußteils 3 mit einer Kanüle vorgesehen ist.

Ferner ist eine Sicherungskappe 1 vorgesehen, die formschlüssig auf das Verschlußteil 3 aufgesetzt ist, dabei das Verschlußteil 3 umschließt und im aufgesetzten Zustand auf dem Verschlußteil 3 gehalten ist.

Im Inneren der Sicherungskappe 1 ist ein elastisches Dichtungsteil 2 angeordnet, das in die zentrale Ausnehmung 10 des Verschlußteils 3 vorsteht und der Oberfläche des Dichtungselements 5 in dem mit 4 bezeichneten Bereich anliegt. Das Aufsetzen der Sicherungskappe 1 erfolgt am Ende des Herstellungs- bzw. Konfektionierungsprozesses noch unter sterilen Bedingungen, so daß sichergestellt ist, daß der von der Ausnehmung 10 im Verschlußteil 3 freigegebene Bereich des Dichtungselements 5 steril ist und von dem elastischen Dichtungsteil 2 steril gehalten wird.

Das Dichtungsteil 2 ist im einzelnen als im wesentlichen zylindrischer Stopfen ausgebildet, der in einem Sackloch der Sicherungskappe 1 angeordnet ist. An seiner zum Dichtungselement 5 weisenden Stirnseite ist das Dichtungsteil 2 mit einer Ringlippe 2.1 versehen, die entlang des Umfanges des Dichtungsteils 2 verläuft. Das Dichtungsteil 2 weist daher im Bereich der Ringlippe 2.1 eine besonders hohe Elastizität auf, wodurch eine hochwertige Abdichtung gewährleistet ist.

An seiner dem Dichtungselement 5 abgewandten Stirnseite weist das Dichtungsteil 2 einen radial vorstehenden Ringflansch 2.2 auf, der in eine Ringnut in der Wand des Sacklochs der Sicherungskappe 1 vorsteht.

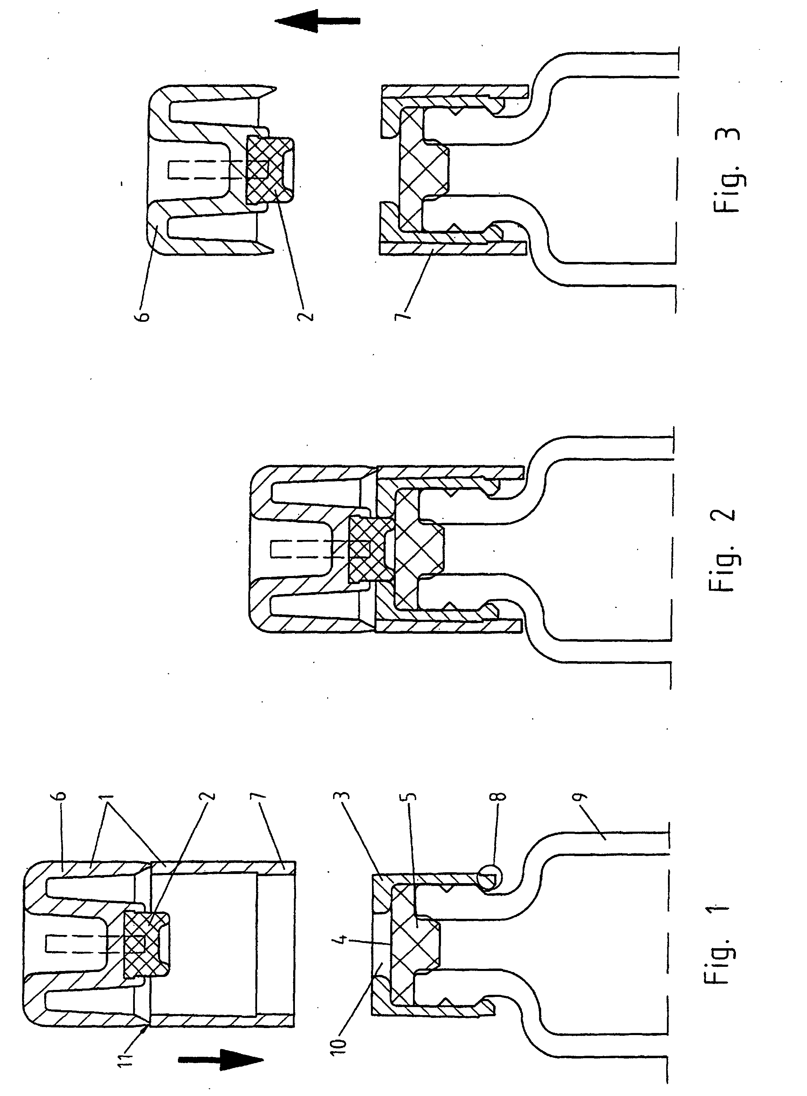

In der ersten Ausführungsform nach den Fig. 1 bis 3 trägt die Sicherungskappe 1 einen Sicherungsring 7, der über eine ringförmig umlaufende Sollbruchzone 11 angeschlossen ist. Dieser Sicherungsring 7 ist über eine Ringschulter 8 im Rastsitz am Verschlußteil 3 gehalten. Nach dem Aufsetzen der Sicherungskappe 1, was durch die Fig. 1 und 2 wiedergegeben wird, läßt sich die Sicherungskappe 1 mit dem Sicherungsringe nicht mehr abziehen. Dadurch ist einerseits sichergestellt, daß das Dichtungsteil 2 nicht unbeabsichtigt vom Dichtungselement 5 abgelöst werden kann, wodurch eine Verschmutzung der zu schützenden Oberfläche 4 ausgeschlossen ist. Zugleich ist hierdurch erkennbar, daß die Spritze mit der fest aufgesetzten Sicherungskappe 1 sich noch in ihrem Originalzustand befindet, also bedenkenlos appliziert werden kann. Hierfür muß der mit 6 bezeichnete Teil der Sicherungskappe 11 entlang der Sollbruchzone abgetrennt werden, wie dies in Fig. 3 dargestellt ist. Hierbei wird das Dichtungsteil 2 vom Dichtungselement 5 abgehoben, das nunmehr über eine sterile Oberfläche 4 verfügt, so daß unmittelbar ohne weitere Maßnahmen mit einer Kanüle durch das Dichtungselement 5 eingestochen werden kann.

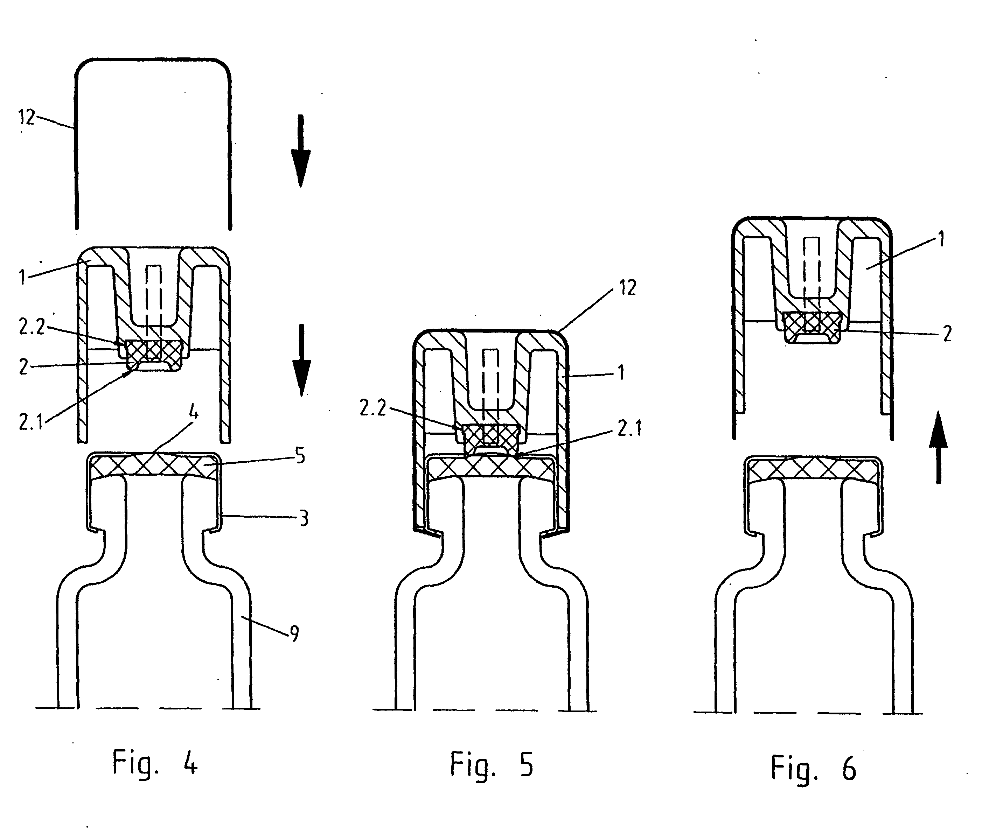

In den Fig. 4 bis 6 ist in einer zweiten Ausführungsvariante eine Karpule dargestellt, bei der das Verschlußteil 3 von einer Bördelkappe, die üblicherweise aus Aluminium besteht, gebildet ist. Bei den marktüblichen Karpulen muß sichergestellt sein, daß der von der Bördelkappe bestimmte Außendurchmesser bei der Applikation nicht vergrößert werden darf, um die Karpule in die gängigen Applikationssysteme einlegen zu können. Daher weist hier die Sicherungskappe 1 keine Sollbruchstelle auf; vielmehr ist die Sicherungskappe von einer Schrumpffolie 12 umhüllt, die die Bördelkappe in ihrem Randbereich mit umschließt. Diese Schrumpffolie 12 dient hier wiederum als "Originalitätsverschluß", läßt also erkennen, ob sich die Karpule noch in ihrem ursprünglichen Zustand befindet.

Nach dem Entfernen der Schrumpffolie 12 läßt sich die Sicherungskappe 1, die hier keinerlei Einrastmittel aufweist, ohne weiteres von der Bördelkappe abziehen, wobei wiederum das Dichtungsteil 2 den Einstechbereich 4 des Dichtungselementes 5 freigibt, der bis dahin von dem Dichtungsteil 2 unter sterilen Bedingungen gehalten wurde.

## Patentansprüche

1. Spritze, insbesondere vorgefüllte Spritze, oder Karpule für medizinische Zwecke, mit einem Spritzenzylinder (9) und gegebenenfalls einem darin angeordneten, mittels einer Kolbenstange verschiebbaren Spritzenkolben, ferner mit einem am kanülenseitigen Ende des Spritzenzylinders (9) axial aufgesetzten Verschlußteil (3), das das kanülenseitige Ende des Spritzenzylinders (9) umgreift, sowie mit einem Dichtungselement (5), das zwischen der Stirnfläche des Spritzenzylinders (9) und dem Verschlußteil (3) angeordnet ist und einerseits stirnseitig dem Spritzenzylinder (9) und andererseits der inneren Stirnfläche des Verschlußteils (3) anliegt, wobei das Verschlußteil (3) mit einer zentralen Ausnehmung (10) versehen ist, sowie mit einer das Verschlußteil (3) umschließenden Sicherungskappe (1), die formschlüssig auf das Verschlußteil (3) aufgesetzt und in aufgesetztem Zustand auf dem Verschlußteil (3) gehalten ist, wobei im Inneren der Sicherungskappe (1) ein elastisches Dichtungsteil (2) angeordnet ist, das in die zentrale Ausnehmung (10) des Verschlußteils (3) vorsteht und der Stimoberfläche des Dichtungselements (5) anliegt, **dadurch gekennzeichnet, daß** das Dichtungsteil (2) als im wesentlichen zylindrischer Stopfen ausgebildet ist, der in einem Sackloch der Sicherungskappe (1) angeordnet und gehalten ist und an seiner zum Dichtungselement (5) weisenden Stirnseite eine entlang des Umfangs verlaufende, den zum Durchstechen mit einer Kanüle vorgesehenen Bereich der zentralen Ausnehmung (10) abdeckende Ringlippe (2.1) aufweist.

2. Spritze oder Karpule nach Anspruch 1, **dadurch gekennzeichnet, daß** das Dichtungsteil (2) an seiner dem Dichtungselement (5) abgewandten Stirnseite einen radial vorstehenden Ringflansch (2.2) aufweist, der in eine Ringnut in der Wand des Sacklochs der Sicherungskappe (1) vorsteht.

3. Spritze oder Karpule nach einem der Ansprüche 1 bis 2, **dadurch gekennzeichnet, daß** die Sicherungskappe (1) einen über eine ringförmig umlaufende Sollbruchzone (11) angeschlossenen Sicherungsring (7) trägt, der über eine Ringschulter (8) im Rastsitz am Verschlußteil (3) gehalten ist.

4. Spritze oder Karpule nach einem der Ansprüche 1 bis 2, bei welcher das Verschlußteil (3) von einer Bördelkappe gebildet ist, **dadurch gekennzeichnet, daß** die Sicherungskappe (1) von einer Schrumpffolie (12) umhüllt ist.

## Claims

1. Syringe, in particular pre-filled syringe, or needle for medicinal purposes, with a syringe cylinder (9) and, if applicable, an injection piston mounted therein, displaceable by means of a piston rod, and in addition surrounded by a closure component (3), axially mounted on the needle side end of the syringe cylinder (9), which surrounds the needle side end of the syringe cylinder (9), together with a sealing element (5), which is mounted between the forepart of the syringe cylinder (9) and the closure component (3) and which on the one side lies against the syringe cylinder (9) on the face and on the other side lies against the internal forepart of the closure component (3), with the closure component (3) being provided with a central recess (10), together with a locking cap (1) surrounding the closure component (3), which is mounted, having positive fit, onto the closure component (3) and is held in the mounted position on the closure component (3), with a flexible sealing component (2) being mounted inside the locking cap (1) which juts out into the central recess (10) of the closure component (3) and lies against the forepart surface of the sealing element (5), distinguished by the fact that the sealing component (2) takes the form of an essentially cylindrical stopper, which is mounted and held in a blind bore of the locking cap (1) and has a ring lip (2.1) on its end face facing towards the sealing element (5), running along the periphery, covering the area of the central recess (10) provided for piercing by a cannula.

2. Syringe or needle as Claim 1, distinguished by the fact that the sealing component (2) has a radially projecting ring flange (2.2) on its end face opposite to the sealing element (5), which juts out into a snap ring groove in the wall of the blind bore of the locking cap (1).

3. Syringe or needle as one of Claims 1 to 2, distinguished by the fact that the locking cap (1) carries a locking ring (7), connected through an annular peripheral predetermined breaking zone (11), the ring being held by a ring shoulder (8) in the fitting seat on the closure component (3).

4. Syringe or needle as one of claims 1 to 2, in which the closure component (3) takes the form of a flanged cap, distinguished by the fact that the locking cap (1) is enveloped by a shrink film (12).

## Revendications

1. Seringue, en particulier seringue préremplie, ou ampoule à usage médical, avec un cylindre de seringue (9) et le cas échéant un piston de seringue qui est disposé à l'intérieur du cylindre de seringue et peut coulisser grâce à une tige de piston, avec en outre un élément formant bouchon (3) qui est enfoncé axialement sur l'extrémité côté canule du cylindre de seringue (9) et entoure ladite extrémité côté canule du cylindre de seringue (9), avec un élément d'étanchéité (5) qui est disposé entre la face frontale du cylindre de seringue (9) et l'élément formant bouchon (3) et est en contact d'une part avec le cylindre de seringue, côté frontal, et d'autre part avec la surface frontale intérieure de l'élément formant bouchon, l'élément formant bouchon (3) comportant une découpe centrale (10), ainsi qu'un capuchon de sécurité (1) qui entoure l'élément formant bouchon (3), est enfilé par complémentarité de formes sur celui-ci (3) et est tenu dans la position enfilée sur l'élément formant bouchon, un élément d'étanchéité élastique (2) étant disposé à l'intérieur du capuchon de sécurité (1), lequel élément d'étanchéité s'avance dans la découpe centrale (10) de l'élément formant bouchon (3) et est en contact avec la surface frontale de l'élément d'étanchéité (5), **caractérisée en ce que** l'élément d'étanchéité (2) est réalisé sous la forme d'un bouchon essentiellement cylindrique, qui est disposé et tenu dans un trou borgne du capuchon de sécurité (1) et présente, sur sa face frontale tournée vers l'élément d'étanchéité (5), une lèvre annulaire (2.1) périphérique qui recouvre la zone destinée à être perforée à l'aide d'une canule de la découpe centrale (10).

2. Seringue ou ampoule selon la revendication 1, **caractérisée en ce que** l'élément d'étanchéité (2), sur sa face frontale éloignée de l'élément d'étanchéité (5), présente une collerette annulaire (2.2) qui fait saillie dans la direction radiale et s'engage dans une rainure annulaire dans la paroi du trou borgne du capuchon de sécurité (1).

3. Seringue ou ampoule selon une des revendications 1 à 2, **caractérisée en ce que** le capuchon de sécurité (1) est liée par une zone destinée à la rupture (11) annulaire à une bague de sécurité (7) qui est maintenue encliquetée sur l'élément formant bouchon (3) grâce à un épaulement annulaire (3).

4. Seringue ou ampoule selon une des revendications 1 à 2, dans laquelle l'élément formant bouchon (3) est formé d'une capsule sertie, **caractérisée en ce que** le capuchon de sécurité (1) est enveloppé d'un film rétractable (12).
